## Europäisches Patentamt

(19) **European Patent Office**

**Office européen des brevets**

(11) Publication number: **0 061 867**
**B1**

(12) **EUROPEAN PATENT SPECIFICATION**

(45) Date of publication of patent specification: **16.09.87**

(21) Application number: **82301367.7**

(22) Date of filing: **17.03.82**

(51) Int. Cl.⁴: **A 61 B 17/10**, A 61 B 17/08

(54) **Staple and cartridge for use in a tissue-stapling device and a tissue-closing method.**

(30) Priority: **27.03.81 US 248575**

(43) Date of publication of application:
**06.10.82 Bulletin 82/40**

(45) Publication of the grant of the patent:
**16.09.87 Bulletin 87/38**

(84) Designated Contracting States:
**CH DE FR GB IT LI SE**

(56) References cited:
**DE-A-2 703 529**
**GB-A-1 198 667**
**GB-A-1 339 394**
**GB-A-2 016 992**
**US-A-4 014 492**
**US-A-4 127 227**

(73) Proprietor: **MINNESOTA MINING AND**
**MANUFACTURING COMPANY**
**3M Center, P.O. Box 33427**
**St. Paul, Minnesota 55133-3427 (US)**

(72) Inventor: **Froehlich, Harold E.**
**2501 Hudson Road P.O. Box 33427**
**St. Paul Minnesota 55133 (US)**

(74) Representative: **Baillie, Iain Cameron et al**
**c/o Ladas & Parry Isartorplatz 5**
**D-8000 München 2 (DE)**

Courier Press, Leamington Spa, England.

## Description

Technical Field

This invention relates to staples, cartridges containing the staples adapted to be incorporated in staple-forming devices.

Background Art

Staples and cartridges containing the staples adapted to be incorporated in the staple-forming devices intended for use to join separated fascia such as during an operation are known in the prior art. One such staple described in U.S. Patent No. 4,014,492 has a straight central portion and pointed L-shaped portions at the ends of the central portion. A staple-forming device closes the staple by bending the junctures of the L-shaped portions at its central portion around the edges of an anvil so that the pointed ends of the L-shaped portions can engage and gather fascia. The closed staple assumes a rectangular shape with the points on the L-shaped portions generally aligned and closely adjacent. Such use of staples can provide much gather (the word "gather" as used herein means the ability of the staple and the device that closes the staple to draw together portions of fascia), which is very desirable so that the edges of the fascia attached will be pressed together to facilitate healing. The rectangular shape of the staple, however, allows the L-shaped legs to be easily bent apart by tension in the gathered fascia so that the holding power of the staple is not as good as may be desired.

U.S. Patent No. 4,127,227 describes a staple and a cartridge containing a plurality of the staples for use in a staple-forming device intended to correct the problem described above. Before it is closed, the staple described in that patent has a straight central portion with curved portions attached at each of its ends, which curved portions have points at their outermost ends adapted to engage fascia. By use of the cartridge in the staple-forming device, the curved end portions are each bent closed to form a closed staple with end loops each engaged only with one portion of the fascia to be joined. The closed loops are generally aligned with the central portion so that tension in the fascia does not act through a large lever arm in the closed loops to tend to open them. While such a closed staple structure minimizes problems with tension in the fascia opening the staple, the staple and its forming process provide very little gather so that fascia fastened by such staples may not be brought together to the degree desired by use of the staple and device. GB—A—1339394 discloses a staple which, when closed, has pointed ends on opposite sides of a central portion, and not in the same plane.

Disclosure of the Invention

The present invention provides a staple, and a cartridge containing the staple adapted to be incorporated in a staple-forming device that will provide both a high degree of gather to ensure approximation of the tissue being joined to promote healing, and a closed staple shape that will strongly resist opening of the staple by tension in the tissue.

According to the present invention there is provided a cartridge, as defined in claim 1, adapted to be incorporated in a staple-forming device for use in joining separated living tissue such as fascia and a staple as defined in claim 3.

Brief Description of Drawing

The present invention will be further described with reference to the accompanying drawing wherein like numerals refer to like parts in the several views, and wherein:

Figure 1 is a plan view of a cartridge according to the present invention;

Figure 2 is an end view of the cartridge shown in Figure 1;

Figure 3 is a sectional view taken approximately along the line 3—3 of Figure 1;

Figures 4 through 6 are enlarged views sequentially showing closing of a staple included in the cartridge of Figure 1;

Figures 7 through 11 are enlarged views sequentially showing the engagement of the staple in the cartridge shown in Figure 1 with portions of living tissue as the staple is closed; and

Figure 12 is an elarged front view of the staple as shown in Figures 5 and 8.

Detailed Description

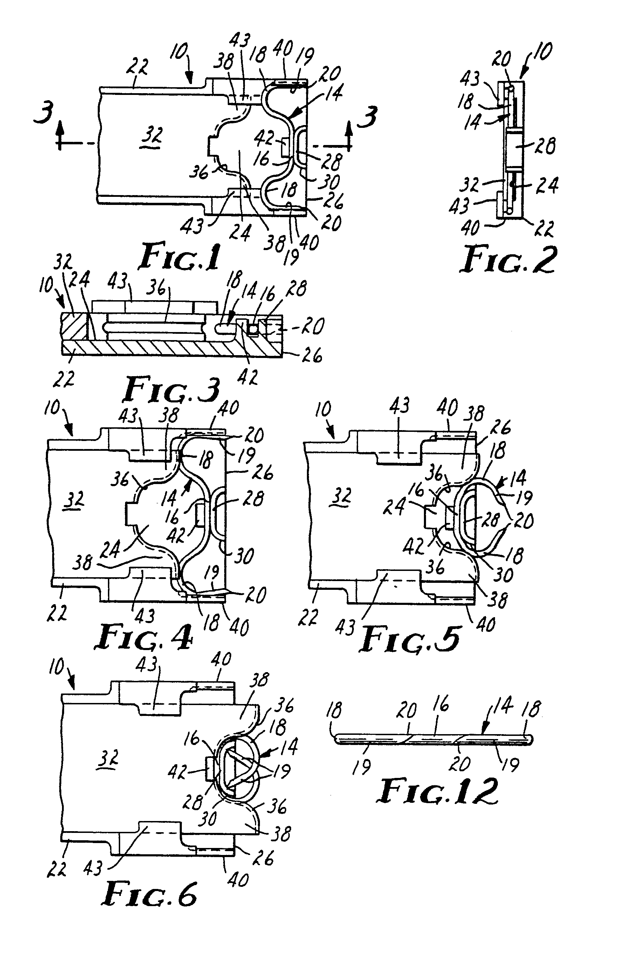

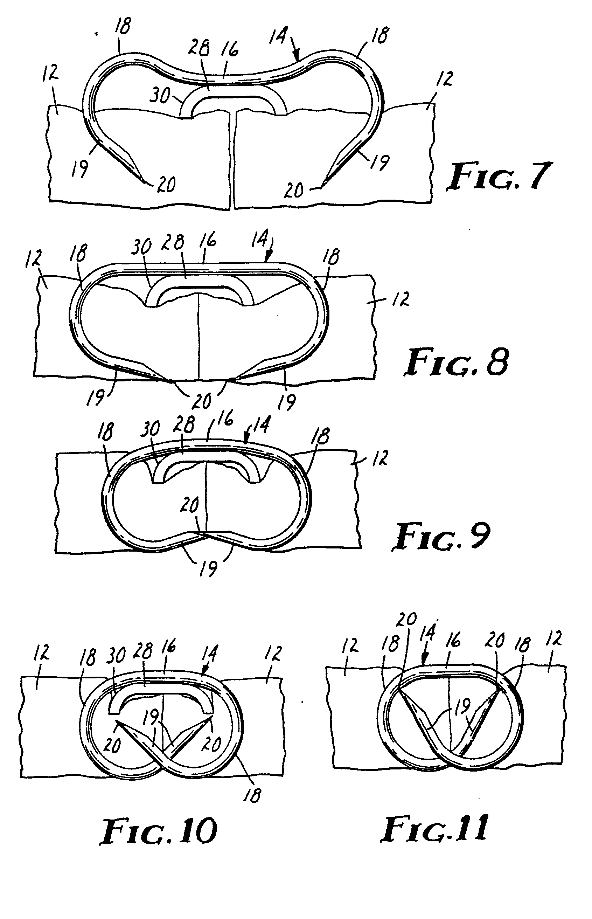

Referring now to Figures 1 through 6 of the drawing, there is shown a cartridge 10 according to the present invention which is adapted to be removably incorporated in a staple-forming device (not shown) intended for use in joining separated living tissue such as fascia 12.

The cartridge 10 comprises at least one open staple 14 comprising a length of wire having a straight or slightly curved central portion 16, and arcuate end portions 18 including straight or slightly curved terminal end parts 19 having points 20 on their ends opposite the central portion 16, with the points 20 on the end portions 18 and the central portion being generally aligned (e.g., within 1.52 mm (0.06 inch) alignment).

The cartridge 10 includes a frame 22 having a planar support surface 24, and means for supporting the staple 14 with its side surfaces parallel to the support surface 24 and the points 20 of the staple 14 adjacent an end 26 of the frame 22, and an anvil 28 projecting at approximately a right angle to the support surface 24 centrally of the staple 14, which anvil 28 has an arcuate surface 30 adapted to be engaged by the central portion 16 and adjacent parts of the end portions 18 of the staple 14. Also included is a ram 32, and means mounting the ram 32 for movement along the planar support surface 24 from a spaced position (Figures 1, 2 and 3) affording placing the open staple 14 against the anvil 28, toward the anvil 28 so that a transversely grooved end surface 36 extending between spaced projections 38 of the ram 32 will engage and bend the staple 14 around the juncture between its central and end portions (Figures 4, 5 and 6). Such bending will form a generally oval shape for the central portion 16 and adjacent parts of the end portions 18 of the staple 14 (Figure 6) and to bring the terminal end parts 19 of the end portions of the staple 14 into side-by-side crossed relationship with the points 20 of each of the end portions 18 positioned adjacent the juncture between the central portion 16 and the other of the end portions 18. By "adjacent the juncture between the central portion 16 and the other of the end portions 18" we mean that the terminal end parts 19 can cross, terminate at (Figure 11), or terminate within (Figure 6) the outer portions of the staple either at or outboard of the juncture; and, particularly with curved terminal end parts 19, could lay approximately along the other of the end portions 18.

The cartridge 10 could be adapted for use in any one of several staple-forming devices (not shown) which afford releasable engagement of the frame 22 of the cartridge 10 with the frame of the device, and which have mechanisms that the ram 32 can be adapted to engage which can be activated either manually or by an electrical or air power supply to provide reciprocal rectilinear movement of the ram 32. One such device is described in U.S. Patent No. 3,949,924.

Also, it is expected that the cartridge could be modified to utilize one of many known types of magazines to feed staples seriatim to the anvil via the operation of the device as each staple at the anvil is engaged with tissue. As illustrated, however, the cartridge 10 contains only the single staple 14 which is initially held in place by means including opposed longitudinally extending grooves in portions of upstanding edge walls 40 on the frame 22, in which grooves the outer surfaces of the terminal end parts 19 of the staple 14 are frictionally held, and a projecting block 42 that engages the central portion 16 of the staple 14 on its side opposite the anvil 28 to prevent it from bowing away from the anvil 28 as the staple 14 is closed.

The means mounting the ram 32 on the frame 22 comprise the support surface 24 along which the ram 32 slides, portions of the upstanding edge walls 40 which guide the edges of the ram 32, and tabs 43 which extend over the ram 32 in spaced relationship to the surface 24 to retain the ram 32 against the surface 24. The transverse groove in the end surface 36 of the ram 32 engages and maintains the staple 14 in alignment as it is formed around the anvil 28. The end surface 36, as viewed along the support surface 24, is generally bell-shaped with a central cavity having a generally oval shape corresponding to the oval shape to be formed in the central portion 16 and adjacent parts of the staple 14, which oval shape of the end surface 30 is interrupted only by a central notch adapted to receive the block 42 as the ram 32 moves to form the staple 14.

Figures 7 through 11 illustrate the engagement of the staple 14 with tissue or fascia 12 and the gather produced by such engagement.

After engagement of points 20 of the staple 14 with the fascia 12, the staple 14 is closed by bending it through almost 180 degrees around the junctures between its central portion 16 and its end portions 18. As the staple enters the fascia 12 (Figure 7), it begins to draw the portions of fascia 12 together and enters the fascia 12 to a predetermined depth controlled by the diameter of the arc in its end portions (18) (Figure 8). Such depth control can be important to prevent piercing of body portions beneath the fascia 12. Subsequently the terminal end parts 19 of the staple 14 will move past each other (Figure 9). Such passing of the terminal end parts 19 is facilitated by shearing the staple 14 from different sides to form the points 20, which, as can be seen in Figure 12, moves the points 20 to opposite sides of the staple 14 so that the points 20 will not meet and the tapered terminal end parts 19 can slide by each other. Each end portion 18 will then enter the portion of fascia 12 through which the other end portion 18 extends (Figure 10) and move to a position with its point 20 adjacent the juncture between the central portion 16 and the other end portion 18 (Figure 11) whereupon the anvil 18 is withdrawn. The resultant closed shape of the staple (Figure 11) will strongly resist opening because tension in the fascia 12 acts through a very short lever arm against the generally oval central portion 16 and adjacent parts of the end portions 18.

Such closure of the staple will gather the tissue 12 by an amount related to the difference in width between the points 20 of the open staple 14 as they enter the tisue 12, and the inner width of the closed staple in which the tissue 12 engaged by the points 20 is contained. Such gather can be expressed as a percentage by dividing that difference in width by the width between the points 20 of the open staple 14. Using such a computation it has been found that the staple 14 closed in the manner illustrated and described above can provide gather in the range of 60 to 70 percent.

**Claims**

1. A cartridge adapted to be incorporated in a staple forming device for closing an opening in living tissue, said cartridge including at least one open staple (14) comprising a length of wire having a central portion (16), and arcuate end portions (18) at opposite ends of the central portion having points (20) at their ends opposite said central portion with the points on said end portions and said central portion being aligned, said end portions (18) of the staple (14) when closed being arranged to extend through the tissue on one side of the closed opening and project into the tissue on the other side of the closed opening; a frame comprising an anvil (28) adapted to engage said central portion; and a ram movably mounted on said frame and having spaced projections and an end surface therebetween adapted to engage said end portions and bend said staple closed around said anvil generally in a single plane, wherein the end surface (36) of said ram (32) and said anvil (28) are shaped to bend the staple (14) around the juncture between said end portions (18) and said central portion (16) to form the central portion (16) and adjacent parts of said end portions (18) into the shape of a single oval when viewed normal to said single plane, and terminal parts (19) of said end portions (18) in side by side relationship, the difference between the inner width dimension of the oval and the dimension between the points (20) of the open staple (14) providing for an amount of gather for the tissue, said terminal parts being arranged to cross at the side of the oval opposite the central portion (16) and project across the oval with the points (20) on each side of the end portions (18) positioned adjacent the juncture between the central portion (16) and the other of the end portions (18).

2. A cartridge according to claim 1 further characterized in that the closing of said staple (14) gathers the tissue in the range of about 60 to 70 percent.

3. A staple lying generally in a single plane and arranged to close an opening through fascia, wherein said staple (14), when closed and when viewed normal to said single plane comprises a single portion (16) and adjacent parts (18) in the shape of a single oval, which adjacent parts bridge between and pierce through adjacent portions of the fascia, and pointed end parts (19) in side by side crossed relationship at the side of the oval opposite the central portion (16) and projecting across the oval so that the points (20) on each of said end parts (19) can project into the fascia and are positioned adjacent the central portion (16) of the staple (14).

**Patentansprüche**

1. Patrone zum Einsetzen in eine Klammerverformungsvorrichtung zum Schließen einer Öffnung in lebendem Gewebe, wobei die Patrone mindestens eine offene Klammer (14) besitzt, die eine Drahtlänge umfaßt, die einen mittleren Teil (16) und an dessen entgegengesetzten Enden angeordnete, bogenförmige Endteile (18) besitzt, die an ihren dem mittleren Teil entgegengesetzten Ende Spitzen (20) besitzen, die allgemein mit dem mittleren Teil fluchten, wobei die Endteile (18) der Klammer (14) in deren geschlossenem Zustand das Gewebe auf der einen Seite der geschlossenen Öffnung durchsetzen und in das Gewebe auf der anderen Seite der geschlossenen Öffnung hineinragen; ferner einen Rahmen, der einen Amboß (28) besitzt, der geeignet ist, an dem mittleren Teil anzugreifen, und einen auf dem Rahmen gelagerten Stößel, der im Abstand voneinander angeordnete Vorsprünge und zwischen diesen eine Stirnfläche zum Angriff an den Endteilen und zum Zusammenbiegen der Klammer um den Amboß allgemein in einer einzigen Ebene aufweist, dadurch gekennzeichnnet, daß die Stirnfläche (36) des Stößels (32) und der Amboß (28) so geformt sind, daß sie die Klammer (14) an den Übergängen zwischen den Endteilen (18) und dem mittleren Teil (16) derart biegen, daß der mittlere Teil (16) und die benachbarten Teile der Endteile (18) bei Betrachtung in einer zu der genannten einzigen Ebene normalen Richtung die Form eines einzigen Ovals annehmen, die Differenz zwischen der lichten Weite des Ovals und dem Abstand zwischen den Spitzen (20) der offenen Klammer (14) ein Zusammenraffen des Gewebes ermöglicht, und die äußeren Endbereiche (19) der Endteile (18) auf der dem mittleren Teil (16) entgegengesetzten Seite des Ovals nebeneinanderliegen und einander kreuzen und derart in das Oval hineinragen, daß die Spitze (20) jedes der beiden Endteile (18) dem Übergang zwischen dem mittleren Teil (16) und dem anderen Endteil (18) benachbart ist.

2. Patrone nach Anspruch 1, dadurch gekennzeichnet, daß durch das Schließen der Klammer (14) das Gewebe um etwa 60 bis 70% zusammengerafft wird.

3. Klammer, die allgemein in einer einzigen Ebene liegt und geeignet ist, eine Öffnung in Faszien zu schließen, dadurch gekennzeichnet, daß die Klammer (14) im geschlossenen Zustand bei Betrachtung in einer zu der einzigen Ebene normalen Richtung einen mittleren Teil (16) und an diesen abschließende Teile (18) in Form eines einzigen Ovals besitzt, die genannten anschließenden Teile einander benachbarte Teile der Faszien verbinden durchstechen, die Klammer ferner spitze Endbereiche (19) besitzt, die auf der dem mittleren Teil (16) entgegengesetzten Seite des Ovals nebeneinanderliegen und einander kreuzen und derart in das Oval hineinragen, daß die Spitzen (20) jedes der Endteile (19) in die Faszien hineinragen kann und dem mittleren Teil (16) der Klammer (14) benachbart ist.

**Revendications**

1. Cartouche prévue pour être incorporée dans un dispositif de formage d'agrafe en vue de fermer une coupure dans un tissu vivant, ladite cartouche contenant au moins une agrafe ouverte (14), constituée d'une longueur de fil comportant une partie centrale (16) et des parties d'extrémité arquées (18) situées aux extrémités opposées de la partie centrale et présentant des pointes (20) à leurs extrémités opposées à ladite partie centrale, les pointes desdites parties d'extrémité et ladite partie centrale étant sensiblement alignées, lesdites parties d'extrémité de l'agrafe (14) lorsqu'elle est fermée étant disposées de manière à s'étendre à travers le tissu sur un côté de la coupure fermée et à pénétrer dans le tissu de l'autre côté de la coupure fermée; un cadre comportant une enclume (28) prévue pour entrer en contact avec ladite partie centrale; et un piston poussoir monté de façon mobile sur ledit cadre et comportant des prolongements espacés et une surface d'extrémité, entre ces derniers, qui peut entrer en contact avec lesdites parties d'extrémité et plier l'agrafe fermée autour de l'enclume sensiblement dans un plan unique; dans laquelle la surface d'extrémité (36) dudit piston (32) et celle de ladite enclume (28) sont profilées de manière à plier l'agrafe (14) au voisinage de la jonction entre lesdites parties d'extrémité (18) et ladite partie centrale (16) pour amener la partie centrale (16) et les régions adjacentes desdites parties d'extrémité (18) dans une configuration ovale unique, vue prependiculairement audit plan unique, la différence entre la largeur intérieure de l'ovale et la dimension entre les pointes (20) de l'agrafe ouverte (14) engendrant une certaine quantité de rapprochement du tissu, et amener les régions terminales (19) desdites parties d'extrémité (18) en relation côté à côté, lesdites régions terminales étant disposées de manière à se croiser du côté de l'ovale opposé à la partie centrale (16) et à se prolonger à travers l'ovale, les pointes (20) de chacune des parties d'extrémité (18) étant situées près de la jonction entre la partie centrale (16) et l'autre des parties d'extrémité (18).

2. Cartouche suivant la revendication 1, caractérisé en outre en ce que la fermeture de ladite agrafe (14) rapproche le tissu, de l'ordre de 60 à 70 % environ.

3. Agrafe s'étendant sensiblement dans un plan unique et prévue pour fermer une coupure à travers un fascia, dans laquelle ladite agrafe (14), lorsqu'elle est fermée et vue perpendiculairement audit plan unique, comprend une partie centrale (16) et des régions adjacentes (18) de configuration ovale unique, les régions adjacentes constituant un pont entre les parties adjacentes du fascia et perçant ces parties, et des régions terminales pointues (19) en relation croisée côté à côté du côté de l'ovale opposé à la partie centrale (16) et se prolongeant à travers l'ovale de sorte que les pointes (20) de chacune desdites régions d'extrémité (19) peuvent se projeter dans le fascia et sont situées près de la partie centrale (16) de l'agrafe (14).

FIG.1

FIG.2

FIG.3

FIG.4

FIG.5

FIG.6

FIG.12

*FIG. 7*

*FIG. 8*

*FIG. 9*

*FIG. 10*

*FIG. 11*